# EUROPEAN PATENT SPECIFICATION

(11) **EP 3 456 848 B1**
(45) Date of publication and mention of the grant of the patent: **04.08.2021**
(21) Application number: 18194007.3
(22) Date of filing: 12.09.2018
(51) Int. Cl.: C14C 1/08

(54) **A DELIMING AGENT AND A USE THEREOF AND A DELIMING PROCESS**
ENTKÄLKUNGSMITTEL UND VERWENDUNG DAVON SOWIE EIN ENTKÄLKUNGSVERFAHREN
AGENT DE DÉCHAULAGE ET SON UTILISATION ET PROCÉDÉ DE DÉCHAULAGE

(30) Priority: 13.09.2017 WO PCT/CN2017/101535
(43) Date of publication of application: 20.03.2019
(73) Proprietor: Stahl International B.V., 5145 PE Waalwijk (NL)
(72) Inventor: He, Li Rong, Pudong, Shanghai 200137 (CN)
(74) Representative: V.O.

(56) References cited:
- DE-A1- 2 049 104
- GB-A- 2 026 538
- US-A1- 2015 376 726
- US-A1- 2016 074 347
- DATABASE WPI Week 201734 Thomson Scientific, London, GB; AN 2017-295236 XP002787849, & CN 106 591 505 A (ANHUI SUOYA DECORATION MATERIAL CO LTD) 26 April 2017 (2017-04-26)
- DATABASE WPI Week 201148 Thomson Scientific, London, GB; AN 2011-F40962 XP002787850, & CN 102 010 917 A (UNIV SHAANXI SCI & TECHNOLOGY) 13 April 2011 (2011-04-13)

## Description

### FIELD OF THE INVENTION

The present invention relates to a leather deliming process using a phenolic compound as deliming agent. The present invention also relates to a use of a phenolic compound as deliming agent.

### DESCRIPTION OF THE RELATED ART

It's well known that in the leather manufacturing process, pretreatments for raw pelts shall be done before tanning and crusting. Liming is one of the pretreatments and a key step to remove hair and unwanted proteins from the pelt and to open up the collagen fiber structure. As a rule, liming is normally used as the vital alkaline agent to achieve graduate and controlled swelling effect. Subsequently, the lime must be removed to get the pelts prepared for further processing. Deliming is about removing calcium hydroxide from the pelt including surfaces and inner parts thereof and lowering pH value of pelts until around 8. The main deliming has hitherto been carried out with acids, acid salts and amides, e.g. phthalic acid, sulfophthalic acid, boric acid, mixture of dicarboxylic acids, polysuccinimide, esters and ammonium salts.

The current deliming system has serious disadvantages in industrial practice. Since the alkalinity in the hides always fluctuates, precise metering of the feeding for deliming agents is hardly possible in mass production. Hence, an overdose can easily happen which results in the dropping of pH to 5. If pH value of the pelts decreases too fast, excessive deliming happens and this leads to surface acid swelling. The consequence of conducting deliming at low pH is the invisible acid swelling of the pelt. This causes the lowering of the tear strength of resulted crust and interferes with subsequent processing such as dyeing. To prevent grain damage and acid swelling, mild deliming should be carried out by applying satisfactory deliming agents which are sufficient in lime removal, and could prevent the deliming at low pH .

Conventional deliming agents used in leather industry are mainly based on ammonium salts, such as ammonium sulfate, ammonium chloride, etc. US 2,318,454, which was granted on May 4, 1943 used ammonium salt as deliming component. Ammonium salts have been widely accepted in leather industry due to the following characteristics: i) cheap raw material prices compared with organic compounds; ii) buffering effect, which provides a narrow pH range in deliming float and prevents the surface damage of the pelts and a mild removal of lime from the limed pelt. As a good buffering agents in deliming recipes, which generate leather with clean surface, even dying property and good handle feeling.

When ammonium salts are used for deliming purpose, they react with calcium hydroxide which is physically and chemically bonded on the limed pelt. For instance, the reactions between ammonium sulfate or ammonium chloride and calcium hydroxide are illustrated as below:

Ca(OH)₂+NH₄Cl → CaCl₂+NH₃+H₂O

Ca(OH)₂+(NH₄)₂SO₄ → CaSO₄+NH₃+H₂O

Ammonium based deliming agents have two shortcomings, which limit their application in industry. The disadvantages are: i) the ammonia released during deliming leads to the workers' exposure in unsafe environment; ii) the high NH₃-N content in tannery effluent makes it necessary to perform NH₃-N removal, which increases the cost and duration of wastewater treatment.

Due to the high NH₃-N pollution induced by ammonium salts, three categories of acids have been explored as deliming agents in literature and patents: i) inorganic acids, like boric acid; ii) small molecular organic acids, including lactic acid, citric acid, succinic acid, or adipic acid; iii) macromolecules, such as polyimides, polysuccinimide and hydroxy polysuccinimide.

Boric acid has very good buffering effect in deliming float and has been used as the alternative deliming agent, which enables even deliming across the thick pelts and generates leather with good quality. However, boric acid has reproductive toxicity and is listed in SVHC (substance of very high concern) according to REACH (Registration, evaluation, authorization and restriction of chemicals). A buffering system which has low impact on workers' health and environment is lacking.

CN 102010917A mentions an ammonia free deliming agent, which is the combination of two organic acids. One organic acid is a small molecular organic acid, such as citric acid, or succinic acid; the other organic acid is gluconic acid or sulfosalicylic acid. However, the above used small molecular organic acids have very limited buffering effect in liming float.

WO 2013/107233 also discloses an ammonia free deliming agent. The deliming agent comprises polysuccinimide or hydroxy polysuccinimide. The deliming of this technology is realized via the route:

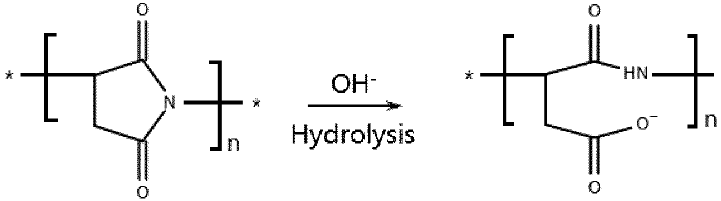

Using polysuccinimide, good deliming effect can be obtained avoiding excessive skin defects by overdosing of deliming chemicals. However, polyimides, polysuccinimides are polymers, which are slow in penetrating into thick limed pelts.

GB 2026538 describes a process for deliming of traditionally limed pelts using esters, which are hydrolyzed during the deliming step by the alkaline components present in the limed pelts. However, esters have a smell and low molecular weight esters are volatile materials and thus contribute to total release of volatile organic compounds (VOC), which is undesirable.

Since the use of ammonium salts, numerous trials have been done to seek for a safe, sufficient and mild deliming agent. However, for many years, environmentally friendly deliming agent which has comparative deliming capacity as ammonium salt and no overdosing problem is required.

### SUMMARY OF THE INVENTION AND ADVANTAGES

Disclosed is a deliming process using a deliming agent comprising phenolic compound with the chemical structure as in Formula I: 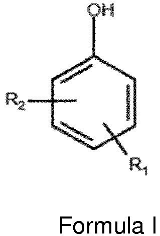 wherein, R₁ is hydroxyl or hydrogen; R₂ is -SO₃⁻M⁺ or -COO⁻M⁺ ; M is alkali metal or alkali earth metal except Ca and Mg.

The deliming agent comprising phenolic compound offers mild deliming process, achieving good deliming effect and crust performance, the Total Kjeldahl nitrogen in effluent being quite low. In another way, the deliming agent avoids dropping the pH value of the deliming solution to less than 5 even with overdosing of deliming agent, thus will not destroy the crust of the pelts and provides soft handle feeling of the crust.

Compared with ammonium based deliming agents, the present invention provides environmentally friendly deliming agents. The present deliming agent has the following features: i) phenolic compound is safe both to environment and workers; ii) phenolic compound will not introduce NH₃-N burden into deliming effluent, and Total Kjeldahl nitrogen in effluent is quite low; iii) the crust performance of pelts is good after deliming.

Compared with the known ammonium free deliming agents, the presently used deliming agent has good deliming effect which is almost equal to that of ammonium salts and will not cause acid swelling.

### DETAILED DESCRIPTION OF THE INVENTION

The object of the present invention is therefore to provide a deliming process using a deliming agent comprising at least one phenolic compound corresponding to the chemical structure in Formula I: 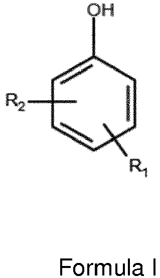 wherein, R₁ is hydroxyl or hydrogen; R₂ is -SO₃⁻M⁺ or -COO⁻M⁺; M is selected from the group consisting of alkali metal and alkali earth metal except Ca and Mg.

In a preferred embodiment, M is selected from the group consisting of Li, Na, K, Rb, Cs, Be, Sr and Ba, more preferably is selected from the group consisting of Li, Na, K and Ba.

The phenolic compound for use in the invention is preferably one or more selected from the group consisting of Formula I-a ∼ Formula I-r, wherein M is Li, Na, K or Ba.

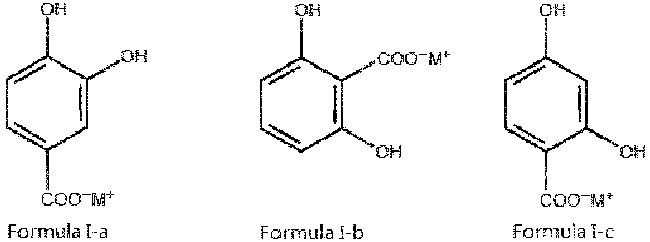

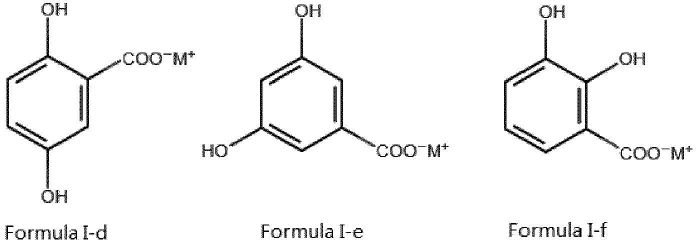

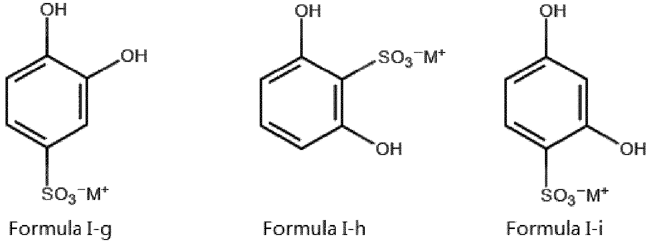

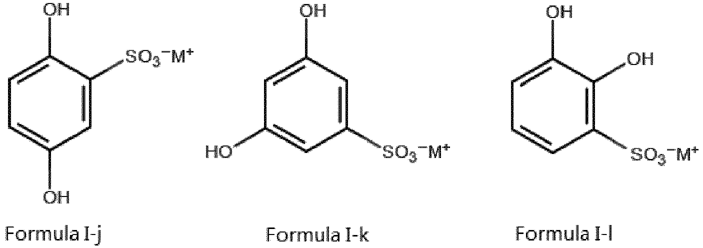

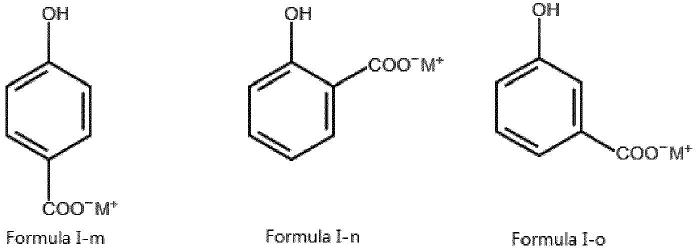

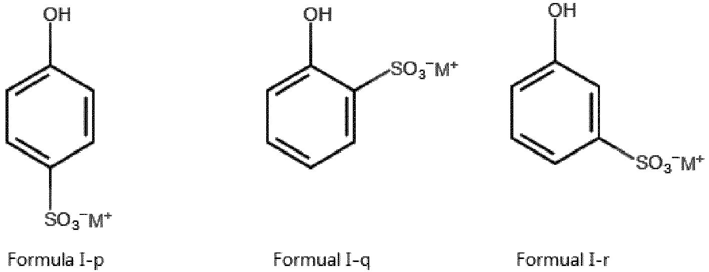

The phenolic compound for use in the invention can further be one or more selected from the group consisting of *p*-hydroxybenzenesulfonic acid sodium salt, *p*- hydroxybenzenesulfonic acid potassium salt, *p*-hydroxybenzenesulfonic acid barium salt, 3-hydroxybenzenesulfonic acid sodium salt, 3-hydroxybenzenesulfonic acid potassium salt, 3-hydroxybenzenesulfonic acid barium salt, 2-hydroxybenzenesulfonic acid sodium salt, 2-hydroxybenzenesulfonic acid potassium salt, 2-hydroxybenzenesulfonic acid barium salt, sodium 4-hydroxybenzoate, potassium 4-hydroxybenzoate, barium 4-hydroxybenzoate, *m*-hydroxybenzoic acid sodium salt, *m-*hydroxybenzoic acid potassium salt, *m*-hydroxybenzoic acid barium salt, sodium salicylate, potassium salicylate, lithium salicylate and barium salicylate.

In a preferred embodiment, the phenolic compound is at least two selected from the group consisting of *p*-hydroxybenzenesulfonic acid sodium salt, *p-*hydroxybenzenesulfonic acid potassium salt, *p*-hydroxybenzenesulfonic acid barium salt, 2-hydroxybenzenesulfonic acid sodium salt, 2-hydroxybenzenesulfonic acid potassium salt, and 2-hydroxybenzenesulfonic acid barium salt.

In a preferred embodiment, the phenolic compound is at least two selected from the group consisting of sodium 4-hydroxybenzoate, potassium 4-hydroxybenzoate, barium 4-hydroxybenzoate, sodium salicylate, potassium salicylate, lithium salicylate and barium salicylate.

The deliming agent to be used in the present invention can further comprise organic acid and/or carbon dioxide. The organic acid which comes into consideration for use in the present invention is one that is commonly used in leather manufacturing process. Preferably, the organic acid comprises at least one selected from the group consisting of lactic acid, citric acid, adipic acid, malonic acid, succinic acid, glutaric acid, and gluconic acid; more preferably is succinic acid and/or adipic acid.

The deliming agent for use in the present invention can further comprise additives. The additives which come into consideration in the present invention is any of the conventional additives used in leather manufacturing industry. The preferable additive is oxidizing agent and/or catalyst. The preferable additive comprises at least one selected from the group consisting of manganese sulfate, manganese chloride, manganese acetate, sodium formate, potassium formate, sodium hydrogen sulfite, sodium metabisulfite, potassium metabisulfite, sodium acetate, potassium acetate, sodium sulfate, potassium sulfate, sodium hydrosulfate, potassium hydrosulfate, sodium percarbonate and potassium percarbonate.

The content of the phenolic compound in the deliming agent is preferably 55-100 wt%, more preferably 70-100 wt%, further more preferably 80-100 wt, wt% is based on the total weight of the deliming agent.

The content of the organic acid in the deliming agent can be chosen according to conventional methods used in leather process industry, preferably is 25 wt% or less; more preferably 5-20 wt%; most preferably 5-10 wt%, wt% is based on the total weight of the deliming agent.

The content of the additive in the deliming agent can be chosen according to conventional methods used in leather process industry, preferably is 25 wt% or less, more preferably 15 wt% or less, most preferably 10 wt% or less, wt% is based on the total weight of the deliming agent.

In a preferred embodiment, the deliming agent comprises 80-100 wt% of the phenolic compound which is at least one of Formula I-m to I-r, 0-10 wt% of the organic acid and 0-10 wt% of the additive, wt% is all based on the total weight of the deliming agent.

The deliming process of the present invention comprises a deliming step: deliming limed pelt with the deliming agent of the present invention.

In the deliming step, the deliming agent is preferably used in the form of an aqueous solution of the deliming agent.

The amount of the water in the aqueous solution can be chosen according to conventional methods used in leather manufacturing process, preferably is 10-300 wt% of the weight of the limed pelt, more preferably 50-200 wt% of the weight of the limed pelt.

The amount of the deliming agent in the deliming step is calculated based on the amount of the phenolic compound, the amount of the phenolic compound is preferably 1- 5 wt%, more preferably 1-3 wt% of the weight of the limed pelt.

The pelt in the present invention could be also explained as hide in the leather manufacturing field. The pelt can come from common mammals used in the leather manufacturing field, such as cattle, sheep, pig, deer, or from common birds.

In an embodiment of the invention, the deliming process comprises: (A) a pre-deliming step: pre-deliming the limed pelt with the additive, the organic acid and/or the carbon dioxide; and (B) the deliming step: deliming the limed pelt with the deliming agent.

The step (A) preferably comprises (i) washing the limed pelt with water, removing the float; (ii) pre-deliming the limed pelt with the additive, and the organic acid and/or carbon dioxide; and (iii) when the pH value of float is about 8, draining the float.

In a preferred embodiment, if the deliming process doesn't comprise the pre-deliming step (A), the deliming step (B) is conducted as deliming the limed pelt in an aqueous solution of the deliming agent.

The organic acid which comes into consideration is one that is commonly used in leather manufacturing processes. Preferably, the organic acid comprises at least one selected from the group consisting of lactic acid, citric acid, adipic acid, malonic acid, succinic acid, glutaric acid, and gluconic acid; more preferably is succinic acid and/or adipic acid.

The additive which comes into consideration is any conventional additive used in leather manufacturing industry. The additive is preferably oxidizing agent and/or catalyst. The preferable additive comprises at least one selected from the group consisting of manganese sulfate, manganese chloride, manganese acetate, sodium formate, potassium formate, sodium hydrogen sulfite, sodium metabisulfite, potassium metabisulfite, sodium acetate, potassium acetate, sodium sulfate, potassium sulfate, sodium hydrosulfate, potassium hydrosulfate, sodium percarbonate and potassium percarbonate.

In step (A), the amount of the additive can be chosen according to conventional methods in the leather manufacturing process, preferably is 0.05-0.3 wt% of the weight of the limed pelt.

In step (A), the amount of the organic acid can be chosen according to conventional methods in the leather manufacturing process, preferably is 0.1-0.5 wt% of the weight of the limed pelt.

The amount of the water in step (i) can be chosen according to conventional methods in leather manufacturing process, preferably is 300 wt% or less, more preferably 200 wt% or less, wt% is based on the weight of the limed pelt.

The deliming step in the present invention can be conducted in the conventional temperature range, preferred is 5 °C-35 °C, more preferred 25 °C-35 °C.

The end of the deliming step can be decided according to conventional methods in leather process industry. Preferably, when the cross section of a cut of the pelt turns to colorless upon the addition of phenolphthalein, and the float of deliming step is 6- 8.8, more preferably is about 8, the deliming step can be ended. The time of the deliming step is preferably from 1 to 6 hours, more preferably from 1 to 3 hours.

The "about 8" in the invention refers to 8 ± 0.8.

During the deliming step, pH value of the float is preferably monitored.

The limed pelt can be limed according to conventional liming methods used in leather process industry.

Another object of the present invention is to provide use of a phenolic compound according to the chemical structure in Formula I as a deliming agent: 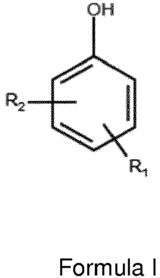 wherein, R₁ is hydroxyl or hydrogen; R₂ is -SO₃⁻M⁺ or -COO⁻M⁺ ; M is alkali metal or alkali earth metal except Ca and Mg.

In a preferred embodiment, M can be preferably selected from the group consisting of Li, Na, K, Rb, Cs, Be, Sr and Ba, more preferably is selected from the group consisting of Li, Na, K and Ba.

The phenolic compound in the invention is preferably one or more selected from the group consisting of Formula I-a ∼ Formula I-r, wherein M is Li, Na, K or Ba.

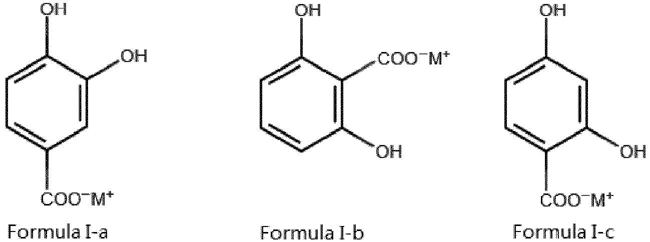

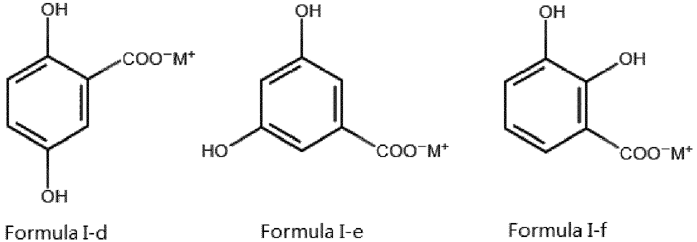

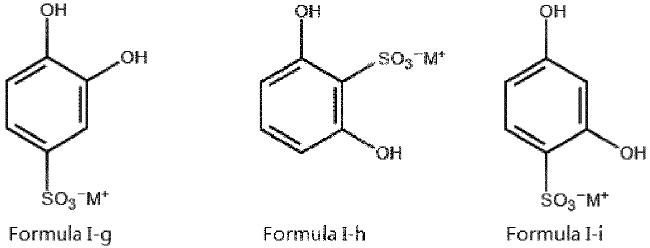

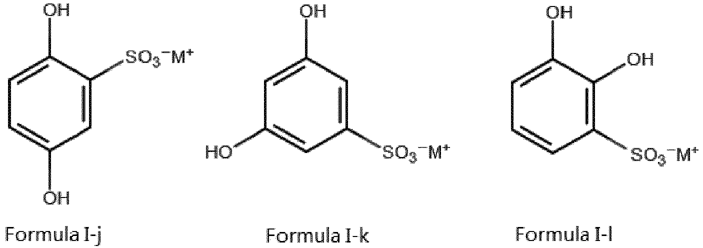

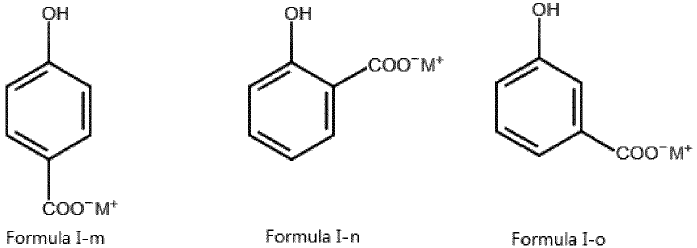

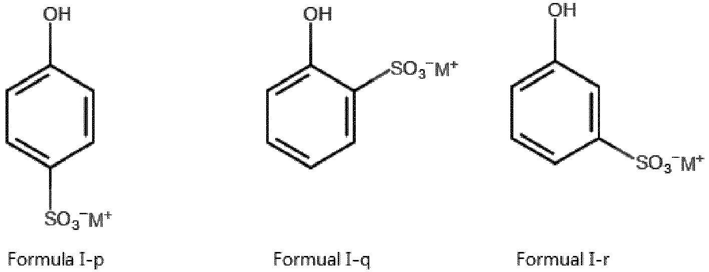

The phenolic compound of the invention can further be one or more selected from the group consisting of *p*-hydroxybenzenesulfonic acid sodium salt, *p-*hydroxybenzenesulfonic acid potassium salt, *p*-hydroxybenzenesulfonic acid barium salt, 3-hydroxybenzenesulfonic acid sodium salt, 3-hydroxybenzenesulfonic acid potassium salt, 3-hydroxybenzenesulfonic acid barium salt, 2-hydroxybenzenesulfonic acid sodium salt, 2-hydroxybenzenesulfonic acid potassium salt, 2-hydroxybenzenesulfonic acid barium salt, sodium 4-hydroxybenzoate, potassium 4-hydroxybenzoate, barium 4-hydroxybenzoate, *m*-hydroxybenzoic acid sodium salt, *m-*hydroxybenzoic acid potassium salt, *m*-hydroxybenzoic acid barium salt, sodium salicylate, potassium salicylate, lithium salicylate and barium salicylate.

In a preferred embodiment, the phenolic compound is at least two selected from the group consisting of *p*-hydroxybenzenesulfonic acid sodium salt, *p-*hydroxybenzenesulfonic acid potassium salt, *p*-hydroxybenzenesulfonic acid barium salt, 2-hydroxybenzenesulfonic acid sodium salt, 2-hydroxybenzenesulfonic acid potassium salt, and 2-hydroxybenzenesulfonic acid barium salt.

In a preferred embodiment, the phenolic compound is at least two selected from the group consisting of sodium 4-hydroxybenzoate, potassium 4-hydroxybenzoate, barium 4-hydroxybenzoate, sodium salicylate, potassium salicylate, lithium salicylate and barium salicylate.

In the present invention, all the technical features mentioned above can be freely combined to form the preferred embodiments.

The present invention has the following benefits: (1) raw material does not contain boric acid, and is compliant with safe operation; (2) without using ammonium salts and greatly reducing the ammonia-nitrogen values in the effluent; (3) no ammonia releasing during deliming process, which facilitates safe operation; (4) avoid dropping the deliming solution pH value to less than 5 even with overdosing of deliming chemicals; (5) the phenolic compound has high level removal efficiency of lime, which enables a clean pelt surface and soft handle feeling of the crust.

### EMBODIMENTS

The following examples serve to illustrate the invention, but they are not intended to limit it thereto:

### Example 1

Cattle hide (thickness 7 mm) which has been soaked and limed in conventional way was used for the application trial. The deliming process comprises the following steps:
(A) Pre-deliming step:
   (i) Limed pelt (thickness 7 mm) was washed in the drum with 150 wt% (wt% is based on the weight of the limed pelt) of water at 30 °C; the float was drained after 10 minutes;
(B) Deliming step:
   (ii) Deliming the limed pelt with an aqueous solution of deliming agent at 30°C, wherein the deliming agent is a mixture of *p*-hydroxybenzenesulfonic acid sodium salt and 2-hydroxybenzenesulfonic acid sodium salt with the mixture molar ratio being 0.45:0.55, the amount of the aqueous solution being 20wt%, the amount of the deliming agent being 2.5wt%, wt% is both based on the weight of the limed pelt;
   (iii) After 120 min, when the cross section of the cut of the pelt gives no color with the addition of phenolphthalein, the pH value of float is 7.8, ending the deliming step, the deliming degree is 100%.

Thereafter, using conventional methods, crust was obtained after bating, pickling, chrome tanning, retanning, neutralization, dying and fat-liquoring.

### Example 2

Cattle hide (thickness 7 mm) which has been soaked and limed in conventional way was used for the application trial. The deliming process comprises the following steps:
(A) Pre-deliming step:
   (i) Limed pelt (thickness 7 mm) was washed in the drum with 150 wt% (wt% is based on the weight of the limed pelt) of water at 30 °C; the float was drained after 10 minutes;
(B) Deliming step:
   (ii) Deliming the limed pelt with an aqueous solution of deliming agent at 30°C, wherein the deliming agent is a mixture of *p*-hydroxybenzenesulfonic acid sodium salt and 2-hydroxybenzenesulfonic acid sodium salt with the mixture molar ratio being 0.45:0.55, the amount of the aqueous solution being 20wt%, the amount of the deliming agent being 5wt%, wt% is both based on the weight of the limed pelt;
   (iii) After 120 min, when the cross section of the cut of the pelt gives no color with the addition of phenolphthalein, the pH value of float is 7.9, ending the deliming step, the deliming degree is 100%.

Thereafter, using conventional methods, crust was obtained after bating, pickling, chrome tanning, retanning, neutralization, dying and fat-liquoring.

### Example 3

Cattle hide (thickness 7 mm) which has been soaked and limed in conventional way was used for the application trial. The deliming process comprises the following steps:
(A) Pre-deliming step:
   (i) Limed pelt (thickness 7 mm) was washed in the drum with 150 wt% (wt% is based on the weight of the limed pelt) of water at 30 °C; the float was drained after 10 minutes;
(B) Deliming step:
   (ii) Deliming the limed pelt with an aqueous solution of deliming agent at 30°C, wherein the deliming agent is sodium 4-hydroxybenzoate, the amount of the aqueous solution is 20wt%, the amount of the deliming agent is 2.5wt%, wt% is both based on the weight of the limed pelt;
   (iii) After 120 min, when the cross section of the cut of the pelt gives no color with the addition of phenolphthalein, the pH value of float is 8, ending the deliming step, the deliming degree is 100%.

Thereafter, using conventional methods, crust was obtained after bating, pickling, chrome tanning, retanning, neutralization, dying and fat-liquoring.

### Example 4

Cattle hide (thickness 7 mm) which has been soaked and limed in conventional way was used for the application trial. The deliming process comprises the following steps:
(A) Pre-deliming step:
   (i) Limed pelt (thickness 7 mm) was washed in the drum with 150 wt% (wt% is based on the weight of the limed pelt) of water at 30 °C; the float was drained after 10 minutes;
(B) Deliming step:
   (ii) Deliming the limed pelt with an aqueous solution of deliming agent at 30°C, wherein the deliming agent is sodium 4-hydroxybenzoate, the amount of the aqueous solution is 20wt%, the amount of the deliming agent is 5wt%, wt% is both based on the weight of the limed pelt;
   (iii) After 120 min, when the cross section of the cut of the pelt gives no color with the addition of phenolphthalein, the pH value of float is 8, ending the deliming step, the deliming degree is 100%.

Thereafter, using the conventional methods, crust was obtained after bating, pickling, chrome tanning, retanning, neutralization, dying and fat-liquoring.

### Comparative example 1

Cattle hide which has been soaked and limed in conventional way was used for the application trial.

The deliming process comprises the following steps:
(A) Pre-deliming step:
   (i) Limed pelt (thickness 7 mm) was washed in the drum with 150 wt% (wt% is based on the weight of the limed pelt) of water at 30 °C; the float was drained after 10 minutes;
(B) Deliming step:
   (ii) Deliming the limed pelt with an aqueous solution of deliming agent at 30°C, wherein the deliming agent is ammonium sulfophthalate, the amount of the aqueous solution is 20wt%, the amount of the deliming agent is 2.5wt%, wt% is both based on the weight of the limed pelt; the pH value of the float and the deliming extent was checked after 30 min, 90 min, 120 min;
   (iii) After 120 min, when the cross section of the cut of the pelt gives no color with the addition of phenolphthalein, the pH value of float is 8.4, ending the deliming step, the deliming degree is 100%.

Thereafter, using the conventional methods, crust was obtained after bating, pickling, chrome tanning, retanning, neutralization, dying and fat-liquoring.

All the floats of example 1-4 and comparative example 1 were taken to check TKN value. The deliming results and the crust effect of each of the examples are shown in Table 1.

**Table 1**

| Examples | Deliming agent | pH | | | Penetration time (min) | TKN | Crust Evaluation |
|---|---|---|---|---|---|---|---|
| | | 30 min | 90 min | 120 min | | | |
| Comp 1 | 2.5 wt% Ammonium sulfophthalate | 4.2 | 7.6 | 8.4 | 120 | 5800 ppm | Rougher grain |
| 1 | 2.5 wt% sodium phenol sulfonate | 7.0 | 7.5 | 7.8 | 120 | 270 ppm | Good |
| 2 | 5 wt% sodium phenol sulfonate | 7.2 | 7.5 | 7.9 | 120 | 300 ppm | Good |
| 3 | 2.5 wt% sodium phenol carboxylate | 6.8 | 7.7 | 8.0 | 120 | 250 ppm | Good |
| 4 | 5 wt% sodium phenol carboxylate | 7.3 | 7.6 | 7.9 | 120 | 320 ppm | Good |

As shown in Table 1, for comparative example 1, after 30 minutes, the pH of the float is 4.2. The pH increased to 8.4 after a further 90 minutes, which indicates that the pH of float can reach a low value even without overdosing of deliming agent. The resulting delimed pelts are not as clean and bright as in examples 1-4. The afforded crust has a rougher grain than the corresponding crusts in examples 1-4. With conventional dosage of sodium phenol sulfonate and sodium phenol carboxylate in example 1 and 3, the pH of float is around 7.0 after 30 minutes. The end pH reaches around 8.0 after 120 minutes. Even under overdosing conditions in examples 2 and 4, the starting pH is around 7.0 and the end pH keeps around 8.0. The deliming is complete and the afforded crust has a fine grain, clean surface and even dyeing effect. In comparison with ammonium sulfophthalate, using the phenol compound as deliming agent decreases the TKN in effluent to only around 5% of the effluent from ammonium salt.

## Claims

1. A deliming process comprising a deliming step: deliming limed pelt with a deliming agent comprising at least one phenolic compound corresponding to the chemical structure in Formula I: 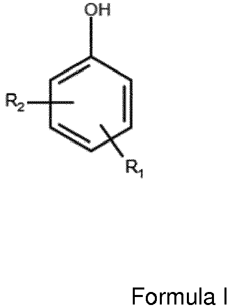 wherein, R₁ is hydroxyl or hydrogen; R₂ is -SO₃⁻M⁺ or -COO⁻M⁺ ; M is selected from the group consisting of alkali metal and alkali earth metal except Ca and Mg.

2. The deliming process according to claim 1, wherein M is selected from the group consisting of Li, Na, K, Rb, Cs, Be, Sr and Ba.

3. The deliming process according to claim 1 or 2, wherein the phenolic compound is one or more selected from the group consisting of Formula I-a ∼ Formula I-r, wherein M is Li, Na, K or Ba;

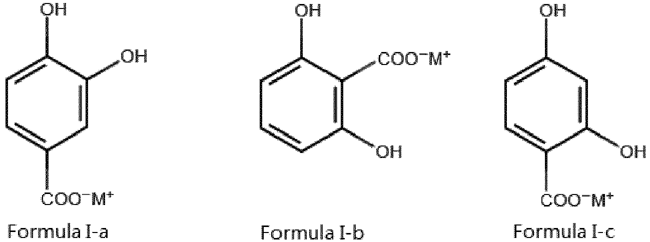

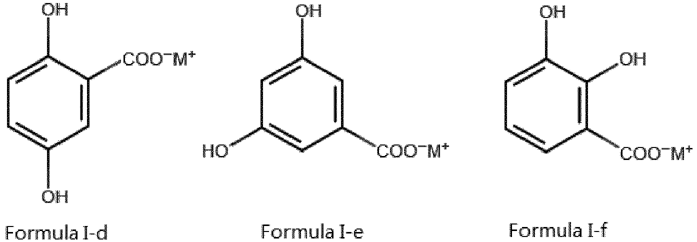

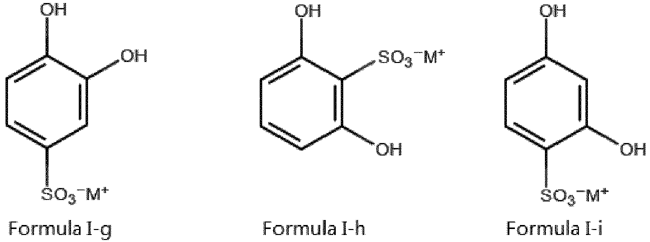

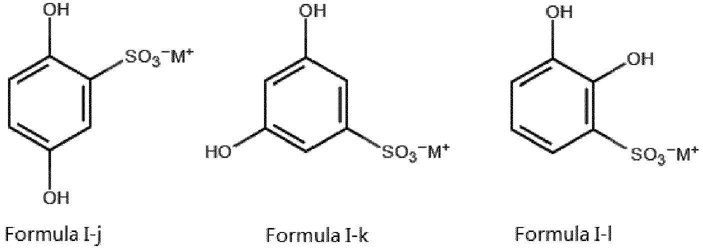

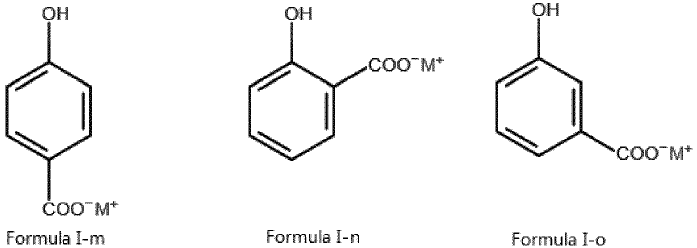

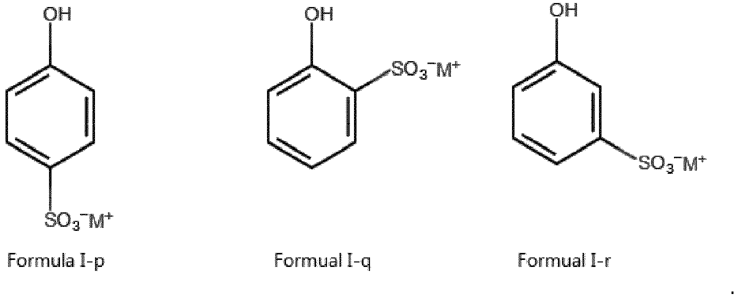

4. The deliming process according to any one of claims 1 to 3, wherein the phenolic compound is one or more selected from the group consisting of *p-*hydroxybenzenesulfonic acid sodium salt, *p*-hydroxybenzenesulfonic acid potassium salt, *p*-hydroxybenzenesulfonic acid barium salt, 3-hydroxybenzenesulfonic acid sodium salt, 3-hydroxybenzenesulfonic acid potassium salt, 3-hydroxybenzenesulfonic acid barium salt, 2-hydroxybenzenesulfonic acid sodium salt, 2-hydroxybenzenesulfonic acid potassium salt, 2-hydroxybenzenesulfonic acid barium salt, sodium 4-hydroxybenzoate, potassium 4-hydroxybenzoate, barium 4-hydroxybenzoate, *m*-hydroxybenzoic acid sodium salt, *m*-hydroxybenzoic acid potassium salt, *m*-hydroxybenzoic acid barium salt, sodium salicylate, potassium salicylate, lithium salicylate and barium salicylate.

5. The deliming process according to any one of claims 1 to 4, wherein the phenolic compound is at least two selected from the group consisting of *p-*hydroxybenzenesulfonic acid sodium salt, p-hydroxybenzenesulfonic acid potassium salt, *p*-hydroxybenzenesulfonic acid barium salt, 2-hydroxybenzenesulfonic acid sodium salt, 2-hydroxybenzenesulfonic acid potassium salt, and 2-hydroxybenzenesulfonic acid barium salt; or at least two selected from the group consisting of sodium 4-hydroxybenzoate, potassium 4-hydroxybenzoate, barium 4-hydroxybenzoate, sodium salicylate, potassium salicylate, lithium salicylate and barium salicylate.

6. The deliming process according to any one of claims 1 to 5, wherein the deliming agent further comprises organic acid and/or carbon dioxide.

7. The deliming process according to claim 6, wherein the organic acid comprises at least one selected from the group consisting of lactic acid, citric acid, adipic acid, malonic acid, succinic acid, glutaric acid, and gluconic acid; preferably is succinic acid and/or adipic acid.

8. The deliming process according to any one of claims 1 to 7, wherein the deliming agent further comprises additive.

9. The deliming process according to claim 8, wherein the additive is oxidizing agent and/or catalyst; preferably additive comprises at least one selected from the group consisting of manganese sulfate, manganese chloride, manganese acetate, sodium formate, potassium formate, sodium hydrogen sulfite, sodium metabisulfite, potassium metabisulfite, sodium acetate, potassium acetate, sodium sulfate, potassium sulfate, sodium hydrosulfate, potassium hydrosulfate, sodium percarbonate and potassium percarbonate.

10. The deliming process according to claim 6 and/or 7, wherein the content of the organic acid in the deliming agent is 25 wt% or less; preferably 5-20 wt%; more preferably 5-10 wt%, wt% is based on the total weight of the deliming agent.

11. The deliming process according to claim 8 and/or 9, wherein the content of the additive in the deliming agent is 25 wt% or less, preferably 15 wt% or less, more preferably 10 wt% or less, wt% is based on the total weight of the deliming agent.

12. The deliming process according to any one of claims 1 to 11, wherein the content of the phenolic compound in the deliming agent is 55-100 wt%, preferably 70-100 wt%, more preferably 80-100 wt%, wt% is based on the total weight of the deliming agent.

13. The deliming process according to any one of claims 1-12, wherein the deliming agent comprises 80-100 wt% of the phenolic compound which is at least one of Formula I-m to I-r, 0-10 wt% of the organic acid and 0-10 wt% of the additive, wt% is all based on the total weight of the deliming agent.

14. The deliming process according to any one of claims 1- 13, wherein the amount of the deliming agent in the deliming step is calculated based on the amount of the phenolic compound, the amount of the phenolic compound is 1- 5 wt%, preferably 1-3 wt% of the weight of the limed pelt.

15. The deliming process according to any one of claims 1-14, wherein the deliming process comprises: (A) a pre-deliming step: pre-deliming the limed pelt with the additive, the organic acid and/or the carbon dioxide; and (B) the deliming step: deliming the limed pelt with the deliming agent.

16. The deliming process according to claim 15, wherein the organic acid comprises at least one selected from the group consisting of lactic acid, citric acid, adipic acid, malonic acid, succinic acid, glutaric acid, and gluconic acid; preferably is succinic acid and/or adipic acid; the additive is oxidizing agent and/or catalyst; preferably additive comprises at least one selected from the group consisting of manganese sulfate, manganese chloride, manganese acetate, sodium formate, potassium formate, sodium hydrogen sulfite, sodium metabisulfite, potassium metabisulfite, sodium acetate, potassium acetate, sodium sulfate, potassium sulfate, sodium hydrosulfate, potassium hydrosulfate, sodium percarbonate and potassium percarbonate.

17. The deliming process according to claim 15 and/or 16, wherein in step (A), the amount of the additive is 0.05-0.3 wt% of the weight of the limed pelt; the amount of the organic acid is 0.1-0.5 wt% of the weight of the limed pelt.

18. Use of phenolic compound corresponding to the chemical structure in Formula I: 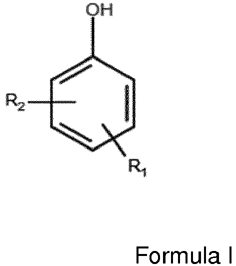 wherein, R₁ is hydroxyl or hydrogen; R₂ is -SO₃⁻M⁺ or -COO⁻M⁺ ; M is selected from the group consisting of alkali metal and alkali earth metal except Ca and Mg as a deliming agent.

## Patentansprüche

1. Entkälkungsverfahren, umfassend einen Entkälkungsschritt:
Entkälken von gekälktem Fell mit einem Entkälkungsmittel, umfassend wenigstens eine phenolische Verbindung entsprechend der chemischen Struktur in Formel I: 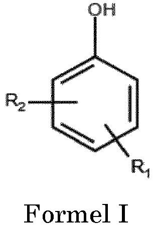 wobei, R1 Hydroxyl oder Wasserstoff ist; R2 -SO₃⁻M⁺ oder -COO⁻M⁺ ist; M ausgewählt ist aus der Gruppe bestehend aus Alkalimetall und Erdalkalimetall außer Ca und Mg.

2. Entkälkungsverfahren nach Anspruch 1, wobei M ausgewählt ist aus der Gruppe bestehend aus Li, Na, K, Rb, Cs, Be, Sr und Ba.

3. Entkälkungsverfahren nach Anspruch 1 oder 2, wobei die phenolische Verbindung eine oder mehrere ist, ausgewählt aus der Gruppe bestehend aus Formel I-a ∼ Formel I-r, wobei M Li, Na, K oder Ba ist;

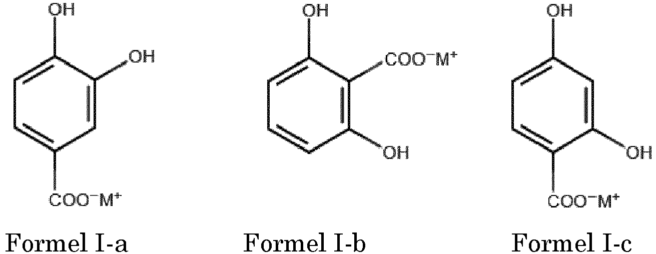

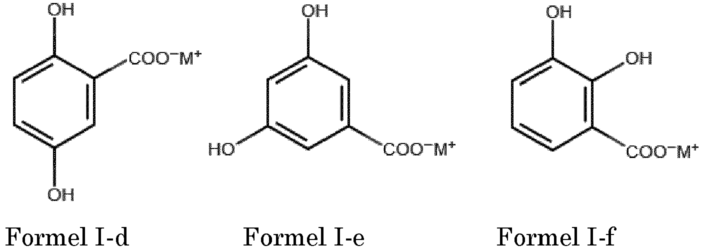

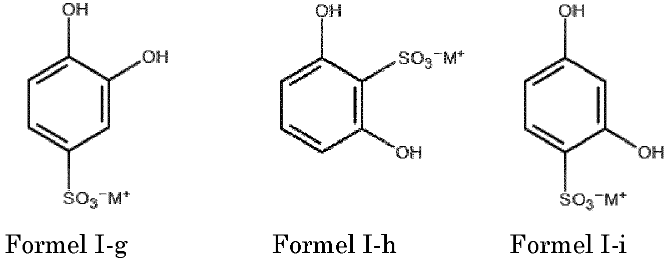

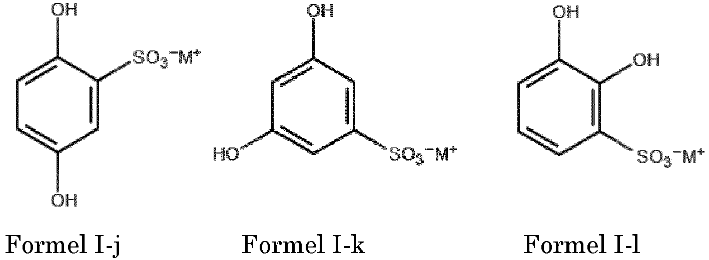

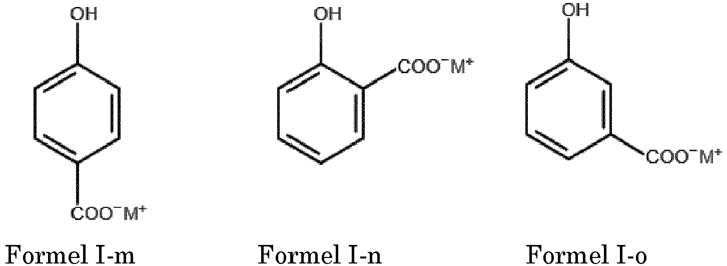

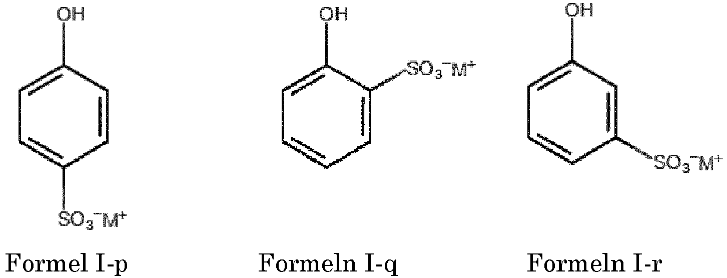

4. Entkälkungsverfahren nach einem der Ansprüche 1 bis 3, wobei die phenolische Verbindung eine oder mehrere ist, ausgewählt aus der Gruppe bestehend aus p-Hydroxybenzolsulfonsäure-Natriumsalz, p-Hydroxybenzolsulfonsäure-Kaliumsalz, p-Hydroxybenzolsulfonsäure-Bariumsalz, 3-Hydroxybenzolsulfonsäure-Natriumsalz, 3-Hydroxybenzolsulfonsäure-Kaliumsalz, 3-Hydroxybenzolsulfonsäure-Bariumsalz, 2-Hydroxybenzolsulfonsäure-Natriumsalz, 2-Hydroxybenzolsulfonsäure-Kaliumsalz, 2-Hydroxybenzolsulfonsäure-Bariumsalz, Natrium-4-Hydroxybenzoat, Kalium-4-Hydroxybenzoat, Barium-4-Hydroxybenzoat, *m*-Hydroxybenzoesäure-Natriumsalz, *m-*Hydroxybenzoesäure-Kahumsalz, *m*-Hydroxybenzoesäure-Bariumsalz, Natriumsalicylat, Kaliumsalicylat, Lithiumsalicylat und Bariumsalicylat.

5. Entkälkungsverfahren nach einem der Ansprüche 1 bis 4, wobei die phenolische Verbindung mindestens zwei ist, ausgewählt aus der Gruppe, bestehend aus *p*-Hydroxybenzolsulfonsäure-Natriumsalz, *p-*Hydroxybenzolsulfonsäure-Kaliumsalz, *p*-Hydroxybenzolsulfonsäure-Bariumsalz, 2-Hydroxybenzolsulfonsäure-Natriumsalz, 2-Hydroxybenzolsulfonsäure-Kaliumsalz, und 2-Hydroxybenzolsulfonsäure-Bariumsalz; oder wenigstens zwei ausgewählt aus der Gruppe bestehend aus Natrium-4-hydroxybenzoat, Kalium-4-hydroxybenzoat, Barium-4-hydroxybenzoat, Natriumsalicylat, Kaliumsalicylat, Lithiumsalicylat und Bariumsalicylat.

6. Entkälkungsverfahren nach einem der Ansprüche 1 bis 5, wobei das Entkälkungsmittel ferner organische Säure und/oder Kohlendioxid umfasst.

7. Entkälkungsverfahren nach Anspruch 6, wobei die organische Säure wenigstens eine umfasst, ausgewählt aus der Gruppe, bestehend aus Milchsäure, Zitronensäure, Adipinsäure, Malonsäure, Bernsteinsäure, Glutarsäure, und Gluconsäure; vorzugsweise Bernsteinsäure und/oder Adipinsäure ist.

8. Entkälkungsverfahren nach einem der Ansprüche 1 bis 7, wobei das Entkälkungsmittel ferner ein Additiv umfasst.

9. Entkälkungsverfahren nach Anspruch 8, wobei das Additiv ein Oxidationsmittel und/oder ein Katalysator ist; vorzugsweise das Additiv wenigstens eines umfasst, ausgewählt aus der Gruppe, bestehend aus Mangansulfat, Manganchlorid, Manganacetat, Natriumformiat, Kaliumformiat, Natriumhydrogensulfit, Natriummetabisulfit, Kaliummetabisulfit, Natriumacetat, Kaliumacetat, Natriumsulfat, Kaliumsulfat, Natriumhydrosulfat, Kaliumhydrosulfat, Natriumpercarbonat und Kaliumpercarbonat.

10. Entkälkungsverfahren nach Anspruch 6 und/oder 7, wobei der Gehalt der organischen Säure in dem Entkälkungsmittel 25 Gew.-% oder weniger ist; vorzugsweise 5-20 Gew.-%; bevorzugter 5-10 Gew.-%, wobei Gew.-% auf das Gesamtgewicht des Entkälkungsmittels bezogen ist.

11. Entkälkungsverfahren nach Anspruch 8 und/oder 9, wobei der Gehalt des Additivs in dem Entkälkungsmittel 25 Gew.-% oder weniger, vorzugsweise 15 Gew.-% oder weniger, noch bevorzugter 10 Gew.-% oder weniger ist, wobei Gew.-% auf das Gesamtgewicht des Entkälkungsmittels bezogen ist.

12. Entkälkungsverfahren nach einem der Ansprüche 1 bis 11, wobei der Gehalt der phenolischen Verbindung in dem Entkälkungsmittel 55-100 Gew.-%, vorzugsweise 70-100 Gew.-%, bevorzugter 80-100 Gew.-% ist, Gew.-% auf das Gesamtgewicht des Entkälkungsmittels bezogen ist.

13. Entkälkungsverfahren nach einem der Ansprüche 1 - 12, wobei das Entkälkungsmittel 80 - 100 Gew.-% der phenolischen Verbindung, die wenigstens eine der Formeln I-m bis I-r ist, 0 - 10 Gew.-% der organischen Säure und 0 - 10 Gew.-% des Additivs umfasst, wobei Gew.-% ganz auf das Gesamtgewicht des Entkälkungsmittels bezogen ist.

14. Entkälkungsverfahren nach einem der Ansprüche 1 - 13, wobei die Menge des Entkälkungsmittels im Entkälkungsschritt bezogen auf die Menge der phenolischen Verbindung berechnet wird, die Menge der phenolischen Verbindung 1 - 5 Gew.-%, vorzugsweise 1 - 3 Gew.-%, des Gewichts des gekälkten Fells ist.

15. Entkälkungsverfahren nach einem der Ansprüche 1 - 14, wobei das Entkälkungsverfahren umfasst: (A) einen Vorentkälkungsschritt:
Vorentkälken des gekälkten Fells mit dem Additiv, der organischen Säure und/oder dem Kohlendioxid; und (B) den Entkälkungsschritt: Entkälken des gekälkten Fells mit dem Entkälkungsmittel.

16. Entkälkungsverfahren nach Anspruch 15, wobei die organische Säure wenigstens eine umfasst, ausgewählt aus der Gruppe, bestehend aus Milchsäure, Zitronensäure, Adipinsäure, Malonsäure, Bernsteinsäure, Glutarsäure, und Gluconsäure; vorzugsweise Bernsteinsäure und/oder Adipinsäure ist; das Additiv Oxidationsmittel und/oder Katalysator ist; vorzugsweise das Additiv wenigstens eines umfasst, ausgewählt aus der Gruppe bestehend aus Mangansulfat, Manganchlorid, Manganacetat, Natriumformiat, Kaliumformiat, Natriumhydrogensulfit, Natriummetabisulfit, Kaliummetabisulfit, Natriumacetat, Kaliumacetat, Natriumsulfat, Kaliumsulfat, Natriumhydrosulfat, Kaliumhydrosulfat, Natriumpercarbonat und Kaliumpercarbonat.

17. Entkälkungsverfahren nach Anspruch 15 und/oder 16, wobei in Schritt (A), die Menge des Additivs 0,05-0,3 Gew.-% des Gewichts des gekälkten Fells ist; die Menge der organischen Säure 0,1-0,5 Gew.-% des Gewichts des gekälkten Fells ist.

18. Verwendung von phenolischer Verbindung entsprechend der chemischen Struktur in Formel I: 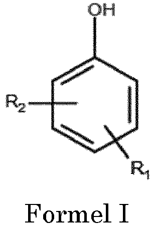 wobei, R1 Hydroxyl oder Wasserstoff ist; R2 -SO₃⁻M⁺ oder -COO⁻M⁺ ist; M ausgewählt ist aus der Gruppe, bestehend aus Alkalimetall und Erdalkalimetall außer Ca und Mg als ein Entkälkungsmittel.

## Revendications

1. Procédé de déchaulage comprenant une étape de déchaulage : déchaulage de la peau chaulée avec un agent de déchaulage comprenant au moins un composé phénolique correspondant à la structure chimique dans la formule I : 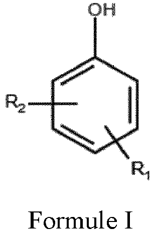 dans laquelle, R1 est hydroxyle ou hydrogène ; R2 est -SO₃⁻M⁺ ou -COO⁻M⁺ ; M est choisi dans le groupe constitué d'un métal alcalin et d'un métal alcalino-terreux à l'exception de Ca et Mg.

2. Procédé de déchaulage selon la revendication 1, dans lequel M est choisi dans le groupe constitué de Li, Na, K, Rb, Cs, Be, Sr et Ba.

3. Procédé de déchaulage selon la revendication 1 ou 2, dans lequel le composé phénolique est l'un ou plusieurs choisis dans le groupe constitué de formule I-a à formule I-r, où M est Li, Na, K ou Ba ;

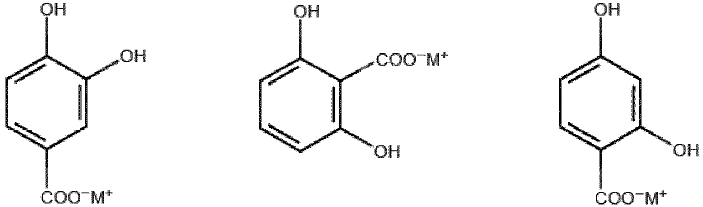

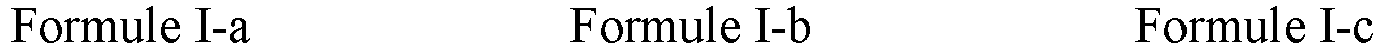

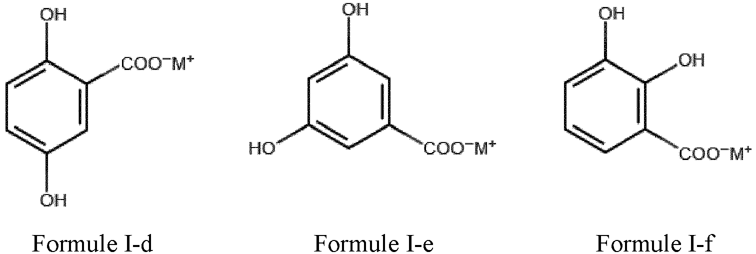

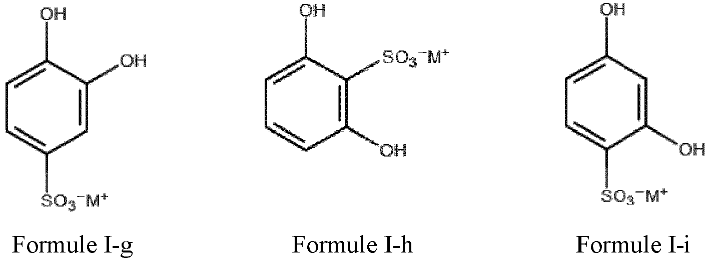

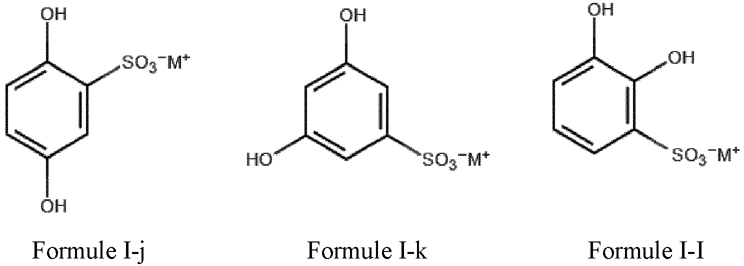

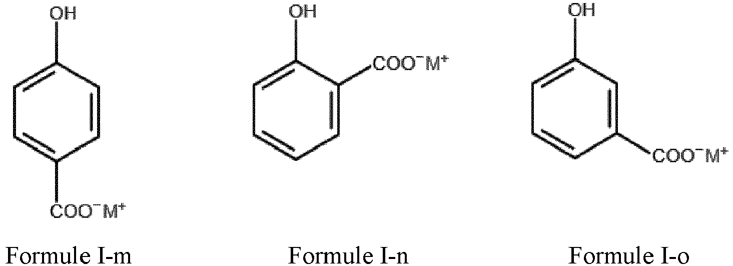

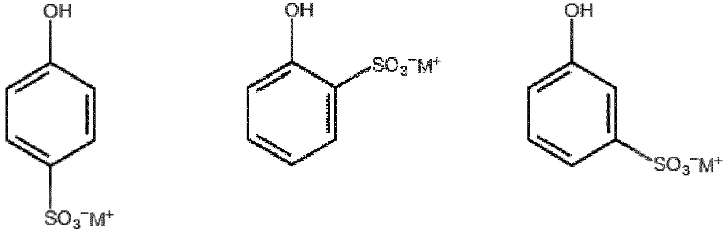

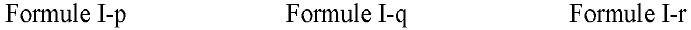

4. Procédé de déchaulage selon l'une quelconque des revendications 1 à 3, dans lequel le composé phénolique est l'un ou plusieurs choisis dans le groupe constitué des sel de sodium d'acide p-hydroxybenzènesulfonique, sel de potassium d'acide p-hydroxybenzènesulfonique, sel de baryum d'acide p-hydroxybenzènesulfonique, sel de sodium d'acide 3-hydroxybenzènesulfonique, sel de potassium d'acide 3-hydroxybenzènesulfonique, sel de baryum d'acide 3-hydroxybenzènesulfonique, sel de sodium d'acide 2-hydroxybenzènesulfonique, sel de potassium d'acide 2-hydroxybenzènesulfonique, sel de baryum d'acide 2-hydroxybenzènesulfonique, 4-hydroxybenzoate de sodium, 4-hydroxybenzoate de potassium, 4-hydroxybenzoate de baryum, sel de sodium d'acide m-hydroxybenzoïque, sel de potassium d'acide m-hydroxybenzoïque, sel de baryum d'acide m-hydroxybenzoïque, salicylate de sodium, salicylate de potassium, salicylate de lithium et salicylate de baryum.

5. Procédé de déchaulage selon l'une quelconque des revendications 1 à 4, dans lequel le composé phénolique est au moins deux choisis dans le groupe constitué des sel de sodium d'acide p-hydroxybenzènesulfonique, sel de potassium d'acide p-hydroxybenzènesulfonique, sel de baryum d'acide p-hydroxybenzènesulfonique, sel de sodium d'acide 2-hydroxybenzènesulfonique, sel de potassium d'acide 2-hydroxybenzènesulfonique et sel de baryum d'acide 2-hydroxybenzènesulfonique ; ou au moins deux choisis dans le groupe constitué des 4-hydroxybenzoate de sodium, 4-hydroxybenzoate de potassium, 4-hydroxybenzoate de baryum, salicylate de sodium, salicylate de potassium, salicylate de lithium et salicylate de baryum.

6. Procédé de déchaulage selon l'une quelconque des revendications 1 à 5, dans lequel l'agent de déchaulage comprend en outre un acide organique et/ou du dioxyde de carbone.

7. Procédé de déchaulage selon la revendication 6, dans lequel l'acide organique comprend au moins l'un choisi dans le groupe constitué des acide lactique, acide citrique, acide adipique, acide malonique, acide succinique, acide glutarique et acide gluconique ; de préférence est l'acide succinique et/ou l'acide adipique.

8. Procédé de déchaulage selon l'une quelconque des revendications 1 à 7, dans lequel l'agent de déchaulage comprend en outre un additif.

9. Procédé de déchaulage selon la revendication 8, dans lequel l'additif est un agent oxydant et/ou un catalyseur ; de préférence l'additif comprend au moins l'un choisi dans le groupe constitué des sulfate de manganèse, chlorure de manganèse, acétate de manganèse, formiate de sodium, formiate de potassium, hydrogénosulfite de sodium, métabisulfite de sodium, métabisulfite de potassium, acétate de sodium, acétate de potassium, sulfate de sodium, sulfate de potassium, hydrosulfate de sodium, hydrosulfate de potassium, percarbonate de sodium et percarbonate de potassium.

10. Procédé de déchaulage selon la revendication 6 et/ou 7, dans lequel la teneur de l'acide organique dans l'agent de déchaulage est de 25 % en poids ou moins ; de préférence 5 à 20 % en poids ; plus préférablement 5 à 10 % en poids, les % en poids étant sur la base du poids total de l'agent de déchaulage.

11. Procédé de déchaulage selon la revendication 8 et/ou 9, dans lequel la teneur de l'additif dans l'agent de déchaulage est de 25 % en poids ou moins, de préférence 15 % en poids ou moins, plus préférablement 10 % en poids ou moins, les % en poids étant sur la base du poids total de l'agent de déchaulage.

12. Procédé de déchaulage selon l'une quelconque des revendications 1 à 11, dans lequel la teneur du composé phénolique dans l'agent de déchaulage est de 55 à 100 % en poids, de préférence 70 à 100 % en poids, plus préférablement 80 à 100 % en poids, les % en poids étant sur la base du poids total de l'agent de déchaulage.

13. Procédé de déchaulage selon l'une quelconque des revendications 1 à 12, dans lequel l'agent de déchaulage comprend 80 à 100 % en poids du composé phénolique qui est au moins l'une parmi les formules I-m à I-r, 0 à 10 % en poids de l'acide organique et 0 à 10 % en poids de l'additif, les % en poids étant tous sur la base du poids total de l'agent de déchaulage.

14. Procédé de déchaulage selon l'une quelconque des revendications 1 à 13, dans lequel la quantité de l'agent de déchaulage dans l'étape de déchaulage est calculée sur la base de la quantité du composé phénolique, la quantité du composé phénolique est de 1 à 5 % en poids, de préférence 1 à 3 % en poids du poids de la peau chaulée.

15. Procédé de déchaulage selon l'une quelconque des revendications 1 à 14, où le procédé de déchaulage comprend : (A) une étape de prédéchaulage : prédéchaulage de la peau chaulée avec l'additif, l'acide organique et/ou le dioxyde de carbone ; et (B) l'étape de déchaulage : déchaulage de la peau chaulée avec l'agent de déchaulage.

16. Procédé de déchaulage selon la revendication 15, dans lequel l'acide organique comprend au moins l'un choisi dans le groupe constitué des acide lactique, acide citrique, acide adipique, acide malonique, acide succinique, acide glutarique et acide gluconique ; de préférence est l'acide succinique et/ou l'acide adipique ; l'additif est un agent oxydant et/ou un catalyseur ; de préférence l'additif comprend au moins l'un choisi dans le groupe constitué des sulfate de manganèse, chlorure de manganèse, acétate de manganèse, formiate de sodium, formiate de potassium, hydrogénosulfite de sodium, métabisulfite de sodium, métabisulfite de potassium, acétate de sodium, acétate de potassium, sulfate de sodium, sulfate de potassium, hydrosulfate de sodium, hydrosulfate de potassium, percarbonate de sodium et percarbonate de potassium.

17. Procédé de déchaulage selon la revendication 15 et/ou 16, dans lequel, dans l'étape (A), la quantité de l'additif est de 0,05 à 0,3 % en poids du poids de la peau chaulée ; la quantité de l'acide organique est de 0,1 à 0,5 % en poids du poids de la peau chaulée.

18. Utilisation du composé phénolique correspondant à la structure chimique dans la formule I : 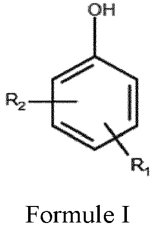 dans laquelle, R1 est hydroxyle ou hydrogène ; R2 est -SO₃⁻M⁺ ou -COO⁻M⁺ ; M est choisi dans le groupe constitué d'un métal alcalin et d'un métal alcalino-terreux à l'exception de Ca et Mg
en tant qu'agent de déchaulage.
